# EUROPEAN PATENT APPLICATION

(11) **EP 2 907 870 A1**
(43) Date of publication of application: **19.08.2015**
(21) Application number: 13844930.1
(22) Date of filing: 09.10.2013
(51) Int. Cl.: C12N 5/10, A61K 35/12, A61K 38/00, A61P 9/00, A61P 25/00, A61P 25/16, C12N 5/071, C07K 14/47

(54) **REPROGRAMMING PEPTIDE AND USE THEREOF**

(30) Priority: 09.10.2012 JP 2012224618
(71) Applicant: Hayashi, Nakanobu, Tokyo 173-0031 (JP)
(72) Inventor: SUGAMA, Kazushige, Takaishi-shi Osaka 592-0002 (JP); WANG, Ke-Yong, Kitakyushu-shi Fukuoka 807-0873 (JP)
(74) Representative: Von Kreisler Selting Werner - Partnerschaft von Patentanwälten und Rechtsanwälten mbB
(86) International application number: PCT/JP2013/077536
(87) International publication number: WO 2014/057997

(57) **Abstract**

The present invention provides a production method of a pluripotent spheroid cell, comprising introducing a reprogramming peptide containing a nuclear localization signal into a differentiated cell; a preparation for induction of a pluripotent spheroid cell from a differentiated cell, which contains the reprogramming peptide, and the like. According to the present invention, a somatic cell can be reprogrammed without using reprogramming factors including transcription factors such as Oct3/4, Sox2 and the like, and low-molecular-weight compounds that promote reprogramming such as HDAC inhibitors and the like, and an induced pluripotent stem cell-like cell, a reprogrammed cell or a transdifferentiated cell can be obtained from a somatic cell.

## Description

### Technical Field

The present invention relates to a pluripotent spheroid cell. Specifically, the present invention relates to a method of producing a pluripotent spheroid cell, comprising introducing a peptide having a nuclear localization signal, i.e., reprogramming peptide, into a differentiated cell. Also, the present invention relates to a differentiated cell obtained by differentiation of a pluripotent spheroid cell and a production method thereof. Furthermore, the present invention relates to a therapeutic agent for a disease associated with a tissue damage, which comprises a reprogramming peptide.

### Background Art

In recent years, iPS cells have been established one after another by reprogramming mouse and human differentiated cells. Takahashi and Yamanaka (non-patent document 1) established an iPS cell by introducing Oct3/4, Sox2, Klf4 and c-Myc genes into a fibroblast derived from a reporter mouse knocked in with a neomycin resistance gene at the Fbx15 gene locus and forcibly expressing same. Okita et al. (non-patent document 2) succeeded in establishing an iPS cell (Nanog iPS cell) showing gene expression and epigenetic modification almost equal to those of embryonic stem (ES) cell by producing a transgenic mouse incorporating green fluorescence protein (GFP) and puromycin resistance gene into the gene locus of Nanog showing localized expression in pluripotent cell rather than Fbx15 forcibly expressing the above-mentioned 4 genes in a fibroblast derived from the mouse, and selecting a puromycin resistant and GFP positive cell. Similar results were also reproduced by other groups (non-patent document 3, non-patent document 4). Thereafter, it was clarified that iPS cell can also be produced by 3 factors excluding c-Myc gene (non-patent document 5).

Furthermore, Takahashi et al. (non-patent document 6) succeeded in establishing,an iPS cell by, introducing 4 genes as in the mouse into a fibroblast derived from human skin. On the other hand, Yu et al. (non-patent document 7) produced human iPS cell by using Oct3/4, Sox2, Nanog and Lin28 genes. As described above, it was shown that an iPS cell having differentiation pluripotency comparable to that of ES cell can be produced in human and mouse by introducing particular factors into a somatic cell.

In the aforementioned Yu et al. (non-patent document 7), iPS cell colony was obtained by 3 kinds of transgenes excluding Nanog or Lin28 gene from the 4 kinds of genes. However, an iPS cell colony could not be obtained by 3 kinds of transgenes excluding Oct3/4 or Sox2 gene, which shows the importance of Oct3/4 and Sox2 genes in establishing an iPS cell (see also patent document 1). Also, Park et al. (non-patent document 8) states that Oct3/4 and Sox2 are essential in establishing an iPS cell.

Furthermore, in patent document 2, a mouse iPS cell was produced by introducing each of Oct3/4, Sox2, Klf4 and c-Myc genes, for which a method including induction of an iPS cell in a Dox-inducible system is disclosed. It is described that an iPS cell could be induced well by using mature B cell as a somatic cell.

Yamanaka (patent document 3) and Thomson et al. (patent document 1) have been disclosed as patent documents relating to the induction of an iPS cell from a somatic cell.

### [Document List]

### [patent documents]

patent document 1: WO 2008/118820
patent document 2: WO 2008/124133
patent document 3: WO 2007/069666

### [non-patent documents]

non-patent document 1: Takahashi, K. and Yamanaka, S., Cell, 126: 663-676 (2006)
non-patent document 2: Okita, K. et al., Nature, 448: 313-317 (2007)
non-patent document 3: Wernig, M. et al., Nature, 448: 318-324 (2007)
non-patent document 4: Maherali, N. et al., Cell Stem Cell, 1: 55-70 (2007)
non-patent document 5: Nakagawa, M. et al., Nat. Biotethnol., 26: 101-106 (2008)
non-patent document 6: Takahashi, K. et al., Cell, 131: 861-872 (2007)
non-patent document 7: Yu, J. et al., Science, 318: 1917-1920 (2007)
non-patent document 8: Park I.H et al., Nature, 451: 141-146 (2008)

### SUMMARY OF THE INVENTION

### Problems to be Solved by the Invention

As mentioned above, the discovery of Yamanaka et al. (Takahashi, K. and Yamanaka, S. Cell, 126: 663-676 (2006)) became the breakthrough, and some combinations have been proposed as reprogramming factors enabling induction of an induced pluripotent stem cell from a somatic cell. In all of them, an induced pluripotent stem cell is induced from a somatic cell. The factor common to the proposed combinations of the reprogramming factors is Oct3/4 alone. While it has recently been reported that reprogramming occurs with Oct3/4 alone (Kim, J.B. et al., Cell, 136: 411-419 (2009)), this is a special case because the somatic cell used was a neural stem cell endogenously expressing Sox2 gene. As a combination of Oct3/4 and other reprogramming factor, combinations of two factors of Oct3/4 and Sox2 (WO 2008/118820), Oct3/4 and Klf4 (Kim, J.B. et al., Nature, 454: 646-650 (2008), Shi, Y. et al., Cell Stem Cell, 2: 525-528 (2008)), and Oct3/4 and c-Myc (Kim, J.B. et al., Nature, 454: 646-650 (2008)) have been reported.

However, it is not clear why the reported combinations of reprogramming factors enable induction of a somatic cell into an induced pluripotent stem cell, and the action mechanism thereof has not been clarified.

Under the circumstances, an object of the present invention is to find a novel method enabling reprogramming of a somatic cell, which has not been reported heretofore, and provide, by using the method, an induced pluripotent stem cell-like cell, a reprogrammed cell or a transdifferentiated cell from a somatic cell.

### Means of Solving the Problems

The present inventors have unexpectedly found this time that pluripotent spheroid cells can be produced from differentiated cells such as somatic cells and the like by introducing a peptide having a nuclear localization signal into the cells, without using 4 factors of Yamanaka including Oct3/4, Sox2 and the like, other reprogramming factors or low-molecular-weight compounds promoting reprogramming such as HDAC inhibitor and the like.

As used herein, the "pluripotent spheroid cell" refers to a cell having spheroid formation capacity and properties of a pluripotent stem cell, which is obtained by introduction of a reprogramming peptide into a differentiated cell. While the properties vary depending on the differentiated cell to be introduced with a nuclear localization signal, the spheroid formed shows positive images of pluripotent stem cell markers of alkaline phosphatase, anti-SSEA-3 antibody, anti-Tra-1-81 antibody, anti-oct3/4 antibody, anti-Sox2 antibody, and anti-Nanog antibody, and presents the state of reprogrammed pluripotency.

Accordingly, the present invention provides the following invention and the like.
[1] A method of producing a pluripotent spheroid cell, comprising introducing a reprogramming peptide into a differentiated cell.
[2] The method of [1], wherein the reprogramming peptide is a peptide having a nuclear localization signal.
[3] The method of [1], wherein the reprogramming peptide is a peptide having a nuclear localization signal and a cell membrane permeation function.
[4] The method of [1], wherein the reprogramming peptide is introduced into the differentiated cell by cultivating the differentiated cell in a medium containing the reprogramming peptide.
[5] The method of [1], wherein the reprogramming peptide is introduced into the differentiated cell by a reverse transfection method.
[6] The method of any of [1] - [5], wherein the pluripotent spheroid is a cell positive to a pericyte marker.
[7] A pluripotent spheroid cell obtained by the method of any of [1] - [6].
[8] A preparation for induction of a pluripotent spheroid cell from a differentiated cell, comprising a reprogramming peptide.
[9] The preparation of [8], wherein the reprogramming peptide is a peptide having a nuclear localization signal.
[10] A pharmaceutical composition comprising the pluripotent spheroid cell of [7].
[11] A therapeutic agent for a disease associated with a tissue damage, which comprises a reprogramming peptide.
[12] A method of treating a disease associated with a tissue damage, comprising administering a reprogramming peptide to a damaged part of the tissue.
[13] A reprogramming peptide for use in the treatment of a disease associated with a tissue damage.
[14] A production method of a differentiated cell, comprising cultivating the pluripotent spheroid cell of [7] in a differentiation medium.
[15] A differentiated cell obtained by the method of [14].

### Effect of the Invention

The pluripotent spheroid cell obtained by the present invention can be differentiated into various cells by cultivation. For example, it can be differentiated into a nervous system cell by continuous culture in a general adhesive incubator, or by culturing in the presence of a differentiation inducer such as retinoic acid, B-27 supplement, bFGF, NGF and the like, whereby, for example, cells positive to neural stem cell markers such as anti-Nestin antibody, cells positive to neuron markers such as anti-neurofilament (NF160) antibody, anti-MAP2 antibody or anti-β Tublin III, and cells positive to glial cell markers such as anti-GFAP antibody are obtained.

The thus-obtained cells can be used for the treatment of diseases such as neurological diseases (cerebral apoplexy, neurodegenerative disease, Parkinson's disease, neurological deficit due to spinal damage) and the like.

When, for example, culture is continued in a cytokine-added low serum medium having a simple composition, cells positive to anti-proinsulin antibody, anti-c-peptide antibody are obtained in about 2 weeks. For the production of an insulin producing β cell, generally, a method including producing an embryoid (EB) from an ES cell or iPS cell, and inducing differentiation in the course of several steps for 1 - 2 months in several kinds of media added with various concentrations of glucose, plural cytokines and a drug is employed. According to the method invented at this time, however, an insulin producing β cell can be produced from a pluripotent spheroid in a high yield in a short period of 1 - 2 weeks.

The thus-obtained cell can be used as autogenic or allogenic insulin-secreting β cell for diabetes treatment, or for screening for a therapeutic drug for diabetes.

Pluripotent spheroid cell shows pluripotency like embryonic stem cells, induced pluripotent stem cell and MUSE cell, and can be differentiated into various kinds of somatic stem cells, progenitor cells, and differentiated cells by cultivating under differentiation culture conditions similar to the conditions for these pluripotent cells. Since pluripotent spheroid cell is obtained by introducing a reprogramming peptide into a differentiated cell, rather than introducing a gene or a high molecular weight physiologically active protein, introduction conditions such as an amount to be introduced and the like can be easily adjusted, and the level of reprogramming of a differentiated cell can be controlled with ease.

### Description of Embodiments

The present invention is explained in more detail.

### 1. definition

The terms used in the present specification encompass the following meanings. Other terms are intended to encompass the meanings generally used in the art.

The pluripotent spheroid cell of the present invention is a cell having pluripotency and the following properties.
(1) Expressing pluripotent markers of Nanog, Oct3/4, SSEA-3, Tra-1-81 and Sox2 and the like.
(2) Having clonality of growing from one cell and continuing to produce own clone.
(3) Having self-renewal ability.
(4) Being capable of in vitro and in vivo differentiation into each of 3 germ layers (endodermal cells, mesodermal cells and ectodermal cells).
(5) Not forming tumor when transplanted to testis or subcutis of mouse.
(6) Becoming positive by alkaline phosphatase staining.
(7) Three dimensional state formed by coagulation of many cells, which changes to a shape of cell spheroid by introduction of a reprogramming peptide into an adherent cell to become a cell having multipotency.

The pluripotent spheroid cell of the present invention is the same as other pluripotent stem cells such as induced pluripotent stem cell and the like in that it is a pluripotent cell obtained by introduction of a reprogramming peptide into a somatic cell, which is a differentiated cell, but is vastly different therefrom in that a pluripotent spheroid cell, which is a reprogrammed cell, can be established in a very short time, and does not form a tumor. The somatic cell, which is a differentiated cell, here refers to any cell derived from a mammal, excluding germ line cells (ovum, oocyte, embryonic stem (ES) cells and the like).

While the pluripotent spheroid cell of the present invention is different from general somatic stem cells of neural stem cells such as bone marrow mesenchymal stem cell, fat-derived mesenchymal stem cell and the like, it has properties similar to those of MUSE cell.

Moreover, the pluripotent spheroid cell of the present invention has the following properties.
(8) Having comparatively moderate proliferation rate, and a division cycle of not less than one day, for example, 1.2 - 1.5 days; however, free of infinite growth shown by ES cell and iPS cell.
(9) Showing asymmetric division during growth.
(10) Having normal karyotype.

Furthermore, the pluripotent spheroid cell of the present invention shows different properties depending on the level of reprogramming thereof and, for example, the pluripotent spheroid cell obtained in the present Examples has differentiation pluripotency, and is positive to both mesenchymal stem cell marker CD105 and pericyte marker CD146.

Representative pluripotent stem cell dispersed in each tissue to be an tissue stem cell includes mesenchymal stem cell MSC and adipose tissue-derived stem cell. Besides these, SKPs (skin-derived progenitor cells) and hair follicle stem cell (HFSC) are also present.

It has recently been considered that pericytes present in the periphery of large and small blood vessels throughout each tissue of the body are the source of pluripotent stem cells such as mesenchymal stem cell, fat-derived stem cell and the like, and are deeply involved in the regeneration and repair of each tissue of the body.

Here, the pluripotent spheroid cell of the present invention being double positive to the both markers of CD105 and CD146 means that a pluripotent spheroid and cell formed by a reprogramming peptide can be utilized as a cell source for regeneration and repair in various tissues such as a clinical application of a mesenchymal stem cell already in the stage of various clinical tests. When it also has the feature of pericyte considered to be the origin of the mesenchymal stem cell, it can be further applied to physiological tissue regeneration.

The term "reprogramming" used in the present specification is also called "nuclear reprogramming", and refers to a process or means for inducing and converting a differentiated cell to an undifferentiated cell, particularly a pluripotent cell. The phenomenon of reprogramming has originally been observed in a method of injecting the nucleus of somatic cell into a denucleated unfertilized egg, fusing an ES cell and a somatic cell, penetrating an ES cell extract in a somatic cell, and the like. Furthermore, reprogramming by introduction of a reprogramming gene or protein such as Oct3/4, Sox2, Klf4, Nanog, c-Myc, Lin28 and the like into a somatic cell has been known. In the reprogramming in the present invention, such complicated operation is not required, and a somatic cell can be reprogrammed into a pluripotent spheroid cell by introducing a reprogramming peptide (reprotin) into a cell.

The "reprogramming peptide" is a peptide that enables such reprogramming. The reprogramming peptide used in the present specification is a peptide containing a nuclear localization signal, and free of the intrinsic function of protein such as a transcriptional regulatory factor showing physiological activity in the nucleus and the like. It is preferably a peptide containing these nuclear localization signals and further having cell membrane-penetrating function. Preferable examples of the reprogramming peptide, as a nuclear localization signal, include nuclear localization signal of SV40 large T antigen, nuclear localization signal of histone 2B, nuclear localization signal of FGF-2 and the like. Examples of the peptide having cell membrane-penetrating function include transmembrane region of HIV gp41 and the like. In addition, reprogramming peptides such as penetratin having the cell membrane-penetrating function and the function of a nuclear localization signal in combination and the like can also be used.

A reprogramming peptide can be introduced by adding same to a medium for cultivating a differentiated cell. A method of adding a reprogramming peptide to a medium may be a method of simply dissolving same in a medium, and a reverse transfection method can also be used. The reverse transfection method is specifically performed by applying a reprogramming peptide to a solid phase surface on which cells are cultivated such as a culture dish and the like and, with or without air-drying, seeding cells thereon. In this way, a reprogramming peptide can be efficiently introduced into a differentiated cell. Since a reprogramming peptide can be introduced into a cell with high efficiency and low toxicity by the reverse transfection method, the amount of a reprogramming peptide to be introduced can be controlled with ease.

When a reprogramming peptide is added to a medium, the concentration thereof varies depending on the culture conditions such as the kind of the reprogramming peptide, the kind of the differentiated cell, the composition of the medium and the like. It is generally added at a concentration of 10 - 200 µg/ml, preferably 20 - 80 µg/ml.

When the reverse transfection method is used, the amount thereof varies depending on the culture conditions such as the kind of the reprogramming peptide, the kind of the differentiated cell, the composition of the medium and the like. It is generally added at a concentration of 1 µg/cm² - 50 µg/cm², preferably 2.5 µg/cm² - 10 µg/cm² per solid phase surface area.

The medium used for producing a pluripotent spheroid cell by introducing a reprogramming peptide into a differentiated cell may be any as long as it is generally suitable for cultivating a differentiated cell.

A somatic cell can be cultured in, for example, a medium obtained by adding substance(s) selected as appropriate such as serum (10% FBS etc.), antibiotic (penicillin, streptomycin etc.), pyruvic acid Na, glutamine, non-essential amino acid, L-dextrose and the like to a basic medium such as DMEM (Dulbecco's modified Eagle medium), MEM (minimum essential medium), α-MEM (minimum essential medium alpha modification), Ham's F12, RPMI1640, a mixed medium thereof and the like, at about 37°C in the presence of 5% CO₂.

When, for example, a reprogramming peptide is introduced into a fibroblast by the reverse transfection method, formation of a pluripotent spheroid occurs highly rapidly from a few hours after the cell seeding and, after mesenchymal-epithelial transition-like morphological change, finally reaches formation of pluripotent spheroid and an increase in pluripotent spheroid by cell proliferation. The pluripotent spheroid increases to have a diameter of 100 - 200 µm, and the pluripotent spheroid formation completes within 2-5 days. It seems at least 30 - 50% of the cells attend to these pluripotent spheroids.

Pluripotent spheroid can be dissociated into single cells by treating with a protease such as trypsin and the like and collagenase and the like. Specifically, for example, dissociation solution for ES/iPS cells (CTK solution: manufactured by ReproCELL) and TrypLE (manufactured by Life Technologies) can be used. The obtained cells can be passaged by cultivating again in a medium, and pluripotent spheroid cells can be proliferated.

Furthermore, the establishment efficiency of pluripotent spheroid cell can be improved by cultivating the cells under low oxygen conditions in an early stage of somatic cell reprogramming (Yoshida, Y. et al., Cell Stem Cell 5: 237-241 (2009)). The term "low oxygen conditions" used in the present specification means that the oxygen concentration in cell culture is considerably low as compared to that of the air. Specific examples of such conditions include an oxygen concentration lower than that of 5 - 10% CO₂/95 -90% air atmosphere, for example, a condition of oxygen concentration of 18% or below. Preferable examples of the oxygen concentration in the aforementioned atmosphere include not more than 15% (e.g., not more than 14%, not more than 13%, not more than 12% or not more than 11%), not more than 10% (e.g., not more than 9%, not more than 8%, not more than 7% or not more than 6%), or 5% not more than (e.g., not more than 4%, not more than 3% or not more than 2%). Alternatively, preferable oxygen concentration in the aforementioned atmosphere is not less than 0.1% (e.g., not less than 0.2%, not less than 0.3% or not less than 0.4%), not less than 0.5% (e.g., not less than 0.6%, not less than 0.7%, not less than 0.8% or not less than 0.95%), or not less than 1% (e.g., not less than 1.1%, not less than 1.2%, not less than 1.3% or not less than 1.4%).

While a method of forming the aforementioned low oxygen state in a cell environment is not limited to the following, the simplest and preferable exemplary method is cultivation of cells in a CO₂ incubator capable of controlling the oxygen concentration. Such incubators are commercially available from various apparatus makers (e.g., Thermo Scientific, Ikemoto Scientific Technology, Juji Field, Wakenyaku and the like) and can be used for the above-mentioned object.

The differentiated cells usable in the present invention are, as defined above, all cells derived from mammal except for germ line cells (ovum, oocyte, embryonic stem (ES) cell and the like) and totipotent cells.

The mammal from which the somatic cell derives is not particularly limited and encompasses any kinds of animals. Preferable mammal is selected from primates (e.g., human, monkey, chimpanzee etc.), rodents (e.g., mouse, rat, hamster, guinea pig etc.), ungulates (bovine, sheep, goat, horse, swine etc.), and pet animals (dog, cat etc.), and more preferable mammals are human and mouse.

The somatic cell non-limitatively encompasses any of somatic cell of fetal period, somatic cell of newborn babies (fetus), and matured somatic cell. In addition, it encompasses any of primary cultured cells, passage cells, and established lines of cells, and further encompasses tissue stem cells and tissue progenitor cells.

Specifically, the somatic cell non-limitatively encompasses, for example, (1) tissue stem cells (somatic stem cells) such as neural stem cell, hematopoietic stem cell, mesenchymal stem cell, dental pulp stem cell and the like, (2) tissue progenitor cells, (3) differentiated cells such as lymphocyte, epithelial cell, endothelial cell, myocyte, fibroblast (dermal cell etc.), hair cell, hepatocyte, stomach mucosa cell, enterocyte, splenocyte, pancreatic cell (pancreatic exocrine cell etc.), brain cell, lung cell, kidney cell, dermal cell and the like, and the like.

A mammal individual suitable as the origin to afford a somatic cell is non-limitatively preferably the patient him/herself, or others having the same or substantially the same HLA type, since rejection does not occur when the obtained pluripotent spheroid cell is used for regenerative medicine. The term "substantially the same" relating to the HLA type used in the present specification means that, when a cell produced by inducing differentiation of a pluripotent spheroid cell is transplanted to a patient, the HLA type matches between the donor and the recipient to the extent permitting survival of the transplanted cell, like when the main HLAs (e.g., gene loci of 3 genes of HLA-A, HLA-B and HLA-DR) are the same. Also, this definition is similarly used in the following. When pluripotent spheroid cell is not administered or transplanted, or when, for example, pluripotent spheroid cell is used as a cell source for screening for the evaluation of the sensitivity and the presence or absence of side effects of medicaments in patients, a somatic cell is desirably obtained from a patient him/herself, or others having the same gene polymorphism correlating to the sensitivity and side effects of the medicament.

Prior to the production of a pluripotent spheroid cell, a somatic cell is obtained from a mammal inclusive of human, cultured in a medium for animal cell culture, and subjected to passage culture if necessary, whereby primary cultured cells or passage cultured cells are obtained. The thus-obtained cultured cells are used for the production of a pluripotent spheroid cell.

Establishment of the pluripotent spheroid cell can be promoted by adding a substance that improves the establishment efficiency of pluripotent spheroid cell to a medium. Such substance includes, for example, cytokines such as basic fibroblast growth factor (bFGF), stem cell factor (SCF) and the like.

In addition, it includes low-molecular-weight compounds, such as histone deacetylase (HDAC) inhibitor, valproic acid (VPA) (Huangfu, D. et al., Nat. Biotechnol., 26(7): 795-797 (2008)), histone methyltransferase (G9a) inhibitor, BIX-01294 (BIX) (Shi, Y. et al., Cell Stem Cell, 2: 525-528 (2008); Kubicek, S. et al., Mol. Cell 25:473-481 (2007); Shi, Y. et al., Cell Stem Cell, 3, 568-574 (2008)), DNA methylating enzyme (Dnmt) inhibitor, 5'-azacytidine (Huangfu, D. et al., mentioned above) and the like. Also, p53 inhibitors such as shRNA siRNA and the like to p53, and UTF1 may be introduced into the cell (Yang Zhao et al., Cell Stem Cell, 3, pp475-479, 2008). Furthermore, the establishment efficiency of pluripotent spheroid cell can also be improved by, in relation to signal transduction, activation of Wnt signal (Marson A. et al., Cell Stem Cell, 3, pp132-135, 2008), inhibition of mitogen-activated protein kinase and glycogen synthase kinase-3 signal transduction (Silva J. et al., PloS Biology, 6, pp2237-2247 2008) and the like, furthermore, introduction of ES cell specific miRNA (e.g., miR-302-367 cluster (Mol. Cell Biol. Doi: 10. 1128/MCB. 00398-08), miR-302 (RNA 14:1-10 (2008)), miR-291-3p, Mir-294 and miR-295 (Nat. Biotechnol. 27: 459-461 (2009)) and the like.

An establishment efficiency improving substance as mentioned above is assumed to have some action on both activation of a gene relating to the differentiation pluripotency and inactivation of a gene specifically expressed in a differentiated cell (Huangfu, D. et al., mentioned above). Also in the method of the present invention, initialization (i.e., reprogramming) of a somatic cell into a pluripotent spheroid cell can be promoted by using an establishment efficiency improving substance.

The pluripotent spheroid cell of the present invention has pluripotency, and can be differentiated into any tissue. The pluripotent spheroid cell can be used for regenerative medicine and the like. For example, the cell can be used for regeneration of various tissues, various organs and the like. Specific examples thereof include nerve, brain spinal cord, liver, muscle, pancreatic islet and the like. The pluripotent spheroid cell of the present invention after administration to a damaged or disordered tissue, organ and the like directly or in the vicinity thereof invades into the tissue and organ, differentiates into a cell specific to the tissue, and can contribute to the regeneration and reconstruction of the tissue and organ. It may also be administered systemically by intravenous administration and the like. In this case, the pluripotent spheroid cell is oriented to, for example, the damaged tissue and organ by homing and the like, reaches there and invades therein, differentiates into the cells of the tissue and organ, and can contribute to the regeneration and reconstruction of the tissue and organ.

Administration can be performed by, for example, parenteral injection such as subcutaneous injection, intravenous injection, muscular injection, intraperitoneal injection and the like, or oral administration, or intrauterus injection to embryo and the like. It may be topical administration or systemic administration. The topical administration can be performed by, for example, utilizing a catheter. The dose can be appropriately determined according to the kind and size of the organ and tissue to be regenerated.

The organ to be regenerated is not limited, and includes bone marrow, spinal cord, blood, spleen, liver, lung, intestine, eye, brain, immune system, circulation system, bone, bond tissue, muscle, heart, blood vessel, pancreas, central nervous system, peripheral nervous system, kidney, bladder, skin, epithelial appendage, breast-mammary gland, adipose tissue, cornea, mucosa including mouth, esophagus, vagina, anus, and the like. In addition, examples of the diseases to be treated include cancer, cardiovascular diseases, metabolism diseases, hepatic diseases, diabetes, hepatitis, hemophilia, diseases of blood system, degenerative or traumatic nerve diseases such as spinal damage and the like, autoimmune diseases, genetic faulty, connective tissue diseases, anemia, infections, transplantation rejection, ischemia, inflammation, damage of skin and muscle and the like.

The cell may be administered together with a substrate acceptable as a medicament. The substrate is made of, for example, a substance having high biological affinity which is made of collagen and the like, or a biodegradable substance, has a shape of particle, plate, tube, container and the like, and the cell only needs to be administered by being bound to the substrate or contained in the substrate.

The pluripotent spheroid cell of the present invention may be induced to differentiate in vitro, a tissue is constructed using the differentiated cell, and the differentiated cell or the tissue may be transplanted. Since the pluripotent spheroid cell of the present invention is free from tumorigenesis, even when the aforementioned differentiated cell or tissue containing the pluripotent spheroid cell of the present invention in an undifferentiated state is transplanted, the possibility of canceration is low and the cell is safe. To avoid rejection of the transplanted cell or tissue by the recipient in these regenerative medicines, a differentiated cell such as mesoderm tissue, mesenchymal tissue and the like is collected from a patient undergoing the regenerative medicine, and the pluripotent spheroid cell of the present invention is desirably produced from the tissue and utilized. Furthermore, the pluripotent spheroid cell of the present invention can be used for the treatment of tissue denaturation and a disease caused by functional disorder. In this case, for example, the pluripotent spheroid cell of the present invention may be concentrated, grown or differentiated ex vivo, and returned to the body. For example, a pluripotent spheroid cell is differentiated into a cell of a particular tissue, and the cell is transplanted to the tissue to be treated. It is also possible to perform an in situ cell treatment by transplanting the cell. In this case, examples of the target cell include liver cell, nerve system cells such as neuron, glial cell and the like, dermal cell, myocytes such as skeletal muscle cell and the like. The pluripotent spheroid cell of the present invention can be differentiated into these cells and transplanted to perform a treatment in situ. By the treatment, for example, Parkinson's disease, cerebral infarction, spinal damage, myodegenerative disease and the like can be treated. Since the pluripotent spheroid cell of the present invention is free from tumorigenesis, the possibility of canceration is low even when it is used for such treatment and the cell is safe.

In addition, the blood and blood components can be formed ex vivo, in vitro by forming blood and blood components by differentiating the pluripotent spheroid cell of the present invention. Examples of the blood component include red blood cell, leukocyte, platelet and the like. The thus-formed blood and blood components can be used for autogenic and allogenic blood transfusions.

As mentioned above, when the pluripotent spheroid cell of the present invention is used for the treatment, it may be differentiated any of ex vivo, in vivo and in vitro. The pluripotent spheroid cell of the present invention differentiates into, for example, osteoblast, chondrocyte, adipocyte, fibroblast, bone marrow stroma, skeletal muscle, smooth muscle, cardiac muscle, eye, endothelium, epithelium, liver, spleen, hematopoiesis, glia, neuron, oligodendrocyte and the like. The pluripotent spheroid cell of the present invention can be differentiated by cultivation in the presence of a differentiation factor. Examples of the differentiation factor include basic fibroblast growth factor (bFGF), vascular endothelial growth factor (VEGF), dimethyl sulfoxide (DMSO) and isoproterenol; or fibroblast growth factor 4 (FGF4), hepatocyte growth factor (HGF) and the like. The present invention also encompasses a cell differentiated from the pluripotent spheroid cell of the present invention.

When the pluripotent spheroid cell of the present invention is used for the treatment, a gene encoding a proteinaceous anti-cancer substance, a physiologically active substance and the like may be introduced. As a result, the pluripotent spheroid cell of the present invention also has a therapeutic drug delivery function. Examples of such substance include anti-angiogenesis drugs.

When a therapeutic agent for a disease associated with a tissue damage, which contains a reprogramming peptide of the present invention, is administered to various tissues, the cells of the tissues partially change into pluripotent spheroid cells, which are differentiated into the cell necessary for each tissue, whereby the tissue can be regenerated. For example, the therapeutic agent can be used for regeneration of various tissues, various organs and the like. Specific examples thereof include nerve, brain spinal cord, liver, muscle, pancreatic islet and the like. The pluripotent spheroid cell of the present invention after administration to a damaged or disordered tissue, organ and the like directly or in the vicinity thereof invades into the tissue and organ, differentiates into a cell specific to the tissue, and can contribute to the regeneration and reconstruction of the tissue and organ.

Administration can be performed by direct administration to a tissue or organ to be regenerated. The dose can be appropriately determined by the kind and size of the organ and tissue to be regenerated.

The organ to be regenerated is not limited, and includes bone marrow, spinal cord, blood, spleen, liver, lung, intestine, eye, brain, immune system, circulation system, bone, bond tissue, muscle, heart, blood vessel, pancreas, central nervous system, peripheral nervous system, kidney, bladder, skin, epithelial appendage, breast-mammary gland, adipose tissue, cornea, mucosa containing mouth, esophagus, vagina, anus, and the like. Examples of the disease to be treated include cancer, cardiovascular diseases, metabolism diseases, hepatic diseases, diabetes, hepatitis, hemophilia, diseases of blood system, degenerative or traumatic nerve diseases such as spinal damage and the like, autoimmune diseases, genetic faulty, connective tissue diseases, anemia, infections, transplant rejection, ischemia, inflammation, damage of skin and muscle, and the like.

The present invention is explained in more detail by the following Examples; however, the scope of the present invention is not limited by the Examples.

### Examples

### Example 1 Preparation of pluripotent spheroid cell by using reprogramming peptide

Human adult fibroblast strain TIG113 was cultured in a medium mainly containing 10% fetal bovine serum-containing EMEM under humidified conditions at 37°C and 5% CO₂ until it reached 1x10⁷ cells. A plate for reverse transfection was prepared by applying each peptide shown in Table 1 to each well of a 12 well plate at a concentration of 30 µg/well, and forming a solid phase by air-drying. Grown TIG113 was washed with CMF-PBS, and incubated in 0.25% trypsin solution at 37°C for 10-15 min to dissociate the cells. Centrifugation and washing with PBS were repeated twice and the cells were suspended in iPS medium (manufactured by ReproCELL Incorporated). Using the cell suspension, human fibroblast was seeded at 2x10⁴ cells on a plate for reverse transfection. As a reverse transfection medium, an iPS medium containing 10% fetal bovine serum and not containing bFGF was used. The number of ES colony-like spheroids formed was measured 7-10 days later, and the spheroid formation degree was evaluated (Table 1). In an experiment using the peptides of H11, H16, and H29, the ratio of the cells forming spheroid was calculated.

As a result of the above-mentioned experiments using various peptides, the following findings were obtained.
1) It has been clarified from the results of H1-H4 experiments that a spheroid formation ability is lost when amino acid of gp41; HIVgp41=GALFLGFLGAAGSTMGA (SEQ ID NO: 21) is mutated. Therefore, HIVgp41 cell membrane permeation function is important for spheroid formation.
2) H13 has no spheroid formation ability. Therefore, spheroid formation does not occur only with the cell membrane permeation function of HIVgp41.
3) Spheroid is not formed by H18 (SV40 Large T antigen NLS) alone. Therefore, spheroid is not formed by nuclear membrane penetrating function alone.
4) When H13 and H18 are simply solid phased together, spheroid is formed. Therefore, simultaneous activation of HIVgp41 peptide and SV40 Large T antigen NLS peptide is important for spheroid formation.
5) H16 (penetratin) having cell membrane-penetrating function and nuclear membrane penetrating function was found to form spheroid by itself.
6) H29 has spheroid formation ability. Therefore, H2B NLS also has spheroid formation ability.
7) The number of cells that join spheroid formation is not less than 80-90% in H11, H16, and H29.

### Conclusion

Spheroid formation activity is found in peptides having cell membrane permeation function and nuclear membrane penetrating function, and a combination of peptides.

**[Table 1-1]**

| peptide No. | characteristics | degree of spheroid formation |
|---|---|---|
| | peptide sequence | |
| H1 | HIVgp41 Linker SV401TaNLS (SEQ ID NO: 1) | ++ |
| | GALFLGFLGAAGSTMGAWSQPKSKRKV | |
| H2 | inactive HIVgp41 Linker SV401TaNLS (SEQ ID NO: 2) | - |
| | GALaLGFLGAAGSTMGAWSQPKSKRKV | |
| H3 | inactive HIVgp41 Linker SV401TaNLS (SEQ ID NO: 3) | - |
| | GALFLGaLGAAGSTMGAWSQPKSKRKV | |
| H4 | inactive HIVgp41 Linker SV401TaNLS (SEQ ID NO: 4) | - |
| | GALaLGaLGAAGSTMGAWSQPKSKRKV | |
| H5 | low active HIVgp41 Linker SV401TaNLS (SEQ ID NO: 5) | + |
| | GALFLGFLGAAGSTMGAmSQPKSKRKV | |
| H6 | stabilizing HIVgp41 Linker SV401TaNLS (SEQ ID NO: 6) | + |
| | Ac-GALFLGFLGAAGSTMGAWSQPKSKRKV-Cysamide | |
| H7 | stabilizing inactive HIVgp41 Linker SV401TaNLS (SEQ ID NO: 7) | - |
| | Ac-GALaLGFLGAAGSTMGAWSQPKSKRKV-Cysamide | |
| H8 | stabilizing inactive HIVgp41 Linker SV401TaNLS (SEQ ID NO: 8) | - |
| | Ac-GALFLGaLGAAGSTMGAWSQPKSKRKV-Cysamide | |
| H9 | stabilizing inactive HIVgp41 Linker SV401TaNLS (SEQ ID NO: 9) | - |
| | Ac-GALaLGaLGAAGSTMGAWSQPKSKRKV-Cysamide | |
| H10 | stabilizing low active HIVgp41 Linker SV401TaNLS (SEQ ID NO: 10) | + |
| | Ac-GALFLGFLGAAGSTMGAmSQPKSKRKV-Cysamide | |
| H11 | HIVgp41 Linker SV401TaNLS (SEQ ID NO: 11) | +++ |
| | Ac-GALFLGFLGAAGSTMGAWSQPKKKRKV-Cysamide | |
| H16 | Penetratin (SEQ ID NO: 12) | ++ |
| | Ac-RQIKIWFQNRRMKWKK-Cysamide | |
| H33 | HIVtat (SEQ ID NO: 13) | - |
| | Ac-GRKKRRQRRRPPQ-Cysamide | |
| H13 | HIVgp41 (SEQ ID NO: 14) | - |
| | Ac-GALFLGFLGAAGSTMGA-Cysamide | |
| H18 | SV401TaNLS (SEQ ID NO: 15) | - |
| | Ac-PKKKRKV-Cysamide | |

**[Table 1-2]**

| | | |
|---|---|---|
| H13+H18 | HIVgp41 + SV401TaNLS | + |
| | Ac-GALFLGFLGAAGSTMGA-Cysamide + Ac-PKKKRKV-Cysamide | |
| H29 | HIVgp41 Linker Histone H2BNLS (SEQ ID NO: 16) | +++ |
| | Ac-GALFLGFLGAAGSTMGAWSQGKKRSKA-Cysamide | |
| H31 | Penetratin Linker Histon H2BNLS(SEQ ID NO: 17) | + |
| | Ac-RQIKIWFQNRRMKWKKWSQGKKRSKA-Cysamide | |
| H32 | Penetratin Linker cMycNLS (SEQ ID NO: 18) | - |
| | Ac-RQIKIWFQNRRMKWKKWSQPAAKRVKLD-Cysamides | |
| H36 | gp41-LIMK2 (SEQ ID NO: 19) | +++ |
| | Ac-GALFLGFLGAAGSTMGA/WSQ/KKRTLRKNDRKKR-Cysamide | |
| H37 | gp41-FGF2 (SEQ ID NO: 20) | +++ |
| | Ac-GALFLGFLGAAGSTMGA/WSQ/RSRKYTSWYVALKR-Cysamide | |

| | | |
|---|---|---|
| Ac shows N-terminus acetylation Cysamide shows C-terminal cysteamide small letter a means alanine having original sequence with mutation small letter m means methionine having original sequence with mutation spheroid formation degree means number of ES cell colony-like spheroids of + about - 10, ++ about 10 - 50, +++ about 50 - 100, - means none. | | |

### Example 2 Characterization of pluripotent spheroid cell

The pluripotent spheroid cell obtained in Example 1, by introducing a reprogramming peptide into differentiated cell, was examined for undifferentiation, expression of nerve marker and the like.

Using the H11 peptide shown in Table 1, the cells were made to form spheroid according to the method shown in Example 1, and incubated in 1% H₂O₂-containing methanol at room temperature for 5 min to immobilize the cells. The cells were washed five times with PBS, and blocked with PBS containing 5% FBS/0.05% gelatin/3% skim milk/1% BSA (hereinafter Block Ace) 5 at room temperature for 1 - 2 hr. Furthermore, the cells were reacted at room temperature for 2 hr with the primary antibody shown in Table 2 and appropriately diluted with Block Ace. The cells were washed five times with PBS, and the HPR-labeled secondary antibody was appropriately diluted with Block Ace and reacted at room temperature for 1 hr. Lastly, the cells were washed five times with PBS, and the antigen was visualized according to the protocol of Peroxidase Stain DAB Kit &enhancer (Nacalai Tesque).

For alkaline phosphatase staining, the cells immobilized by the aforementioned method were incubated at room temperature for 30 min using BCIP/NBT Color Development Substrate (Promega). The reaction was quenched by washing five times with PBS.

### Results

As a result, it has been confirmed that the cells express pluripotent markers alkaline phosphatase, Oct3/4, Sox2, Nanog, SSEA-3, and Tra-1-81. When the culture period becomes long, expression of nestin which is a neural stem cell marker, neurofilament 160, βIII tubulin, MAP2, and GFAP, which are neuron markers, was confirmed. Furthermore, expression of proinsulin and C-peptide, which are insulin producing cell markers, has been confirmed.

### Conclusion

The spheroid formation cell is a novel cell since no ) known cell exists that corresponds thereto in the expression of various markers.

**Table 2**

| antigen | sales company | catalog number |
|---|---|---|
| SSEA-3 | R & D Systems | MAB1434 |
| TRA-1-81antigen | Abcam | ab16289 |
| nestin | Sigma-Aldrich | N5413 |
| βIII tubulin | R & D Systems | MAB1195 |
| neurofilament 160 | Sigma-Aldrich | N5264 |
| Oct3/4 | Funakoshi Corporation | NB100-2379 |
| Sox2 | Funakoshi Corporation | NB110-37235 |
| Nanog | COSMO BIO co., ltd. | RCAB0001P |
| Map2 | AS ONE Corporation | BK-2034-09 |
| GFAP | AS ONE Corporation | BK-2034-08 |
| proinsulin | Abcam | ab8301 |
| c-peptide | Cell Signaling Technology | 4593 |
| Alpha-fetoprotein | NICHIREI BIOSCIENCES INC. | 422211 |
| albumin | NIPPON BIOTEST LABO. | 301-06221 |

### Example 3 Preparation of pluripotent spheroid cell by using human adult fibroblast

Using a method similar to that of Example 1, H11 peptide was introduced into human fibroblast (manufactured by KURABO INDUSTRIES LTD.). The cells showed epithelium-mesenchymal cell transdifferentiation-like changes, and finally formed spheroid.

The prepared spheroid was subjected to expression analysis of pluripotency marker and differentiation marker according to the method of Example 2. As a result, it was positive to pluripotency markers of alkaline phosphatase, SSEA-3, TRA-1-61, Oct3/4, Sox2, and Nanog. Furthermore, the expression of neural stem cell marker nestin, nerve marker neurofilament 160, βIII tubulin, MAP2, and GFAP was confirmed by continuous culture. Furthermore, the expression of insulin producing cell marker proinsulin, and C-peptide was also confirmed.

In addition, the expression of alpha-fetoprotein and albumin was also confirmed. Furthermore, it was also stained by the oil red O staining method, and differentiation into adipose tissue was found.

### Example 4 Preparation of pluripotent spheroid cell by using various cells

By a method similar to that of Example 1, H11 peptide was introduced into BHK21, A549, H293 cell, mouse feeder cell (COSMO BIO co., ltd.: RCHEFC003), bovine vascular endothelial cell (TOYOBO CO., LTD.: CAB300K05), and swine vascular endothelial cell (TOYOBO CO., LTD.: CAP300K05). All cells formed spheroids, and alkaline phosphatase, SSEA-3 positive cells were obtained.

### Example 5 Characterization of pluripotent spheroid cell

The pluripotent spheroid cell obtained in Example 1 by introducing a reprogramming peptide into a differentiated cell was examined for undifferentiation, and expressions of nerve marker and the like.

Using the H11 peptide shown in Table 1, the cells were made to form spheroid according to the method shown in Example 1 and incubated using 1% H₂O₂-containing methanol at room temperature for 5 min, and immobilized.

Using the antibody described in Table 3, cells were stained according to a package insert. As the secondary antibody, HRP-labeled goat anti-mouse IgG antibody (manufactured by Promega KK, Goat Anti-Mouse IgG (H+L), HRP Conjugate) was used.

As a result, the cells were positive to both mesenchymal stem cell marker CD105 and pericyte Pericytes marker CD146. The cells were negative to vascular endothelial cell marker CD31.

**Table 3**

| gene | maker | product name |
|---|---|---|
| 1 | eBioscience | Anti-Human CD105 (Endoglin) |
| 2 | eBioscience | Anti-Human CD146 |
| 3 | eBioscience | Anti-Human CD31 (PECAM-1) |

### Industrial Applicability

The pluripotent spheroid cell of the present invention can be produced from any differentiated cell, like induced pluripotent stem cell, and can be obtained by a convenient method in a shorter period than other pluripotent stem cells such as induced pluripotent stem cell and the like. As compared to other pluripotent stem cells, it can be differentiated into various somatic cells and tissues in a short period, and free from tumor formation. Therefore, it can be an extremely useful cell source in the field of regenerative medicine and drug discovery.

This application is based on a patent application No. 2012-224618 filed in Japan on October 9, 2012, the contents of which are incorporated in full herein.

## Claims

1. A method of producing a pluripotent spheroid cell, comprising introducing a reprogramming peptide into a differentiated cell.

2. The method according to claim 1, wherein the reprogramming peptide is a peptide having a nuclear localization signal.

3. The method according to claim 1, wherein the reprogramming peptide is a peptide having a nuclear localization signal and a cell membrane permeation function.

4. The method according to claim 1, wherein the reprogramming peptide is introduced into the differentiated cell by cultivating the differentiated cell in a medium containing the reprogramming peptide.

5. The method according to claim 1, wherein the reprogramming peptide is introduced into the differentiated cell by a reverse transfection method.

6. The method according to any one of claims 1 to 5, wherein the pluripotent spheroid is a cell positive to a pericyte marker.

7. A pluripotent spheroid cell obtained by the method according to any one of claims 1 to 6.

8. A preparation for induction of a pluripotent spheroid cell from a differentiated cell, comprising a reprogramming peptide.

9. The preparation according to claim 8, wherein the reprogramming peptide is a peptide having a nuclear localization signal.

10. A pharmaceutical composition comprising the pluripotent spheroid cell according to claim 7.

11. A therapeutic agent for a disease associated with a tissue damage, which comprises a reprogramming peptide.

12. A method of treating a disease associated with a tissue damage, comprising administering a reprogramming peptide to a damaged part of the tissue.

13. A reprogramming peptide for use in the treatment of a disease associated with a tissue damage.

14. A production method of a differentiated cell, comprising cultivating the pluripotent spheroid cell according to claim 7 in a differentiation medium.

15. A differentiated cell obtained by the method according to claim 14.


SEQUENCE LISTING

<110>  HAYASHI, Nakanobu

<120>  Reprogramming Peptide and Use Thereof

<130>  092089

<150>  JP 2012-224618
<151>  2012-10-09

<160>  21

<210>  1
<211>  27
<212>  PRT
<213>  Artificial

<220>
<223>  Reprogramming peptide

<400>  1
Gly Ala Leu Phe Leu Gly Phe Leu Gly Ala Ala Gly Ser Thr Met Gly
1               5                   10                  15

Ala Trp Ser Gln Pro Lys Ser Lys Arg Lys Val
            20                  25

<210>  2
<211>  27
<212>  PRT
<213>  Artificial

<220>
<223>  Reprogramming peptide

<400>  2
Gly Ala Leu Ala Leu Gly Phe Leu Gly Ala Ala Gly Ser Thr Met Gly
1               5                   10                  15

Ala Trp Ser Gln Pro Lys Ser Lys Arg Lys Val
            20                  25

<210>  3
<211>  27
<212>  PRT
<213>  Artificial

<220>
<223>  Reprogramming peptide

<400>  3
Gly Ala Leu Phe Leu Gly Ala Leu Gly Ala Ala Gly Ser Thr Met Gly
1               5                   10                  15

Ala Trp Ser Gln Pro Lys Ser Lys Arg Lys Val
            20                  25

<210>  4
<211>  27
<212>  PRT
<213>  Artificial

<220>
<223>  Reprogramming peptide

<400>  4
Gly Ala Leu Ala Leu Gly Ala Leu Gly Ala Ala Gly Ser Thr Met Gly
1               5                   10                  15

Ala Trp Ser Gln Pro Lys Ser Lys Arg Lys Val
            20                  25

<210>  5
<211>  27
<212>  PRT
<213>  Artificial

<220>
<223>  Reprogramming peptide

<400>  5
Gly Ala Leu Phe Leu Gly Phe Leu Gly Ala Ala Gly Ser Thr Met Gly
1               5                   10                  15

Ala Met Ser Gln Pro Lys Ser Lys Arg Lys Val
            20                  25

<210>  6
<211>  27
<212>  PRT
<213>  Artificial

<220>
<223>  Reprogramming peptide

<400>  6
Gly Ala Leu Phe Leu Gly Phe Leu Gly Ala Ala Gly Ser Thr Met Gly
1               5                   10                  15

Ala Trp Ser Gln Pro Lys Ser Lys Arg Lys Val
            20                  25

<210>  7
<211>  27
<212>  PRT
<213>  Artificial

<220>
<223>  Reprogramming peptide

<400>  7
Gly Ala Leu Ala Leu Gly Phe Leu Gly Ala Ala Gly Ser Thr Met Gly
1               5                   10                  15

Ala Trp Ser Gln Pro Lys Ser Lys Arg Lys Val
            20                  25

<210>  8
<211>  27
<212>  PRT
<213>  Artificial

<220>
<223>  Reprogramming peptide

<400>  8
Gly Ala Leu Phe Leu Gly Ala Leu Gly Ala Ala Gly Ser Thr Met Gly
1               5                   10                  15

Ala Trp Ser Gln Pro Lys Ser Lys Arg Lys Val
            20                  25

<210>  9
<211>  27
<212>  PRT
<213>  Artificial

<220>
<223>  Reprogramming peptide

<400>  9
Gly Ala Leu Ala Leu Gly Ala Leu Gly Ala Ala Gly Ser Thr Met Gly
1               5                   10                  15

Ala Trp Ser Gln Pro Lys Ser Lys Arg Lys Val
            20                  25

<210>  10
<211>  27
<212>  PRT
<213>  Artificial

<220>
<223>  Reprogramming peptide

<400>  10
Gly Ala Leu Phe Leu Gly Phe Leu Gly Ala Ala Gly Ser Thr Met Gly
1               5                   10                  15

Ala Met Ser Gln Pro Lys Ser Lys Arg Lys Val
            20                  25

<210>  11
<211>  27
<212>  PRT
<213>  Artificial

<220>
<223>  Reprogramming peptide

<400>  11
Gly Ala Leu Phe Leu Gly Phe Leu Gly Ala Ala Gly Ser Thr Met Gly
1               5                   10                  15

Ala Trp Ser Gln Pro Lys Lys Lys Arg Lys Val
            20                  25

<210>  12
<211>  16
<212>  PRT
<213>  Artificial

<220>
<223>  Reprogramming peptide

<400>  12
Arg Gln Ile Lys Ile Trp Phe Gln Asn Arg Arg Met Lys Trp Lys Lys
1               5                   10                  15

<210>  13
<211>  13
<212>  PRT
<213>  Artificial

<220>
<223>  Reprogramming peptide

<400>  13
Gly Arg Lys Lys Arg Arg Gln Arg Arg Arg Pro Pro Gln
1               5                   10

<210>  14
<211>  17
<212>  PRT
<213>  Artificial

<220>
<223>  Reprogramming peptide

<400>  14
Gly Ala Leu Phe Leu Gly Phe Leu Gly Ala Ala Gly Ser Thr Met Gly
1               5                   10                  15

Ala

<210>  15
<211>  7
<212>  PRT
<213>  Artificial

<220>
<223>  Reprogramming peptide

<400>  15
Pro Lys Lys Lys Arg Lys Val
1               5

<210>  16
<211>  27
<212>  PRT
<213>  Artificial

<220>
<223>  Reprogramming peptide

<400>  16
Gly Ala Leu Phe Leu Gly Phe Leu Gly Ala Ala Gly Ser Thr Met Gly
1               5                   10                  15

Ala Trp Ser Gln Gly Lys Lys Arg Ser Lys Ala
            20                  25

<210>  17
<211>  26
<212>  PRT
<213>  Artificial

<220>
<223>  Reprogramming peptide

<400>  17

Arg Gln Ile Lys Ile Trp Phe Gln Asn Arg Arg Met Lys Trp Lys Lys
1               5                   10                  15

Trp Ser Gln Gly Lys Lys Arg Ser Lys Ala
            20                  25

<210>  18
<211>  28
<212>  PRT
<213>  Artificial

<220>
<223>  Reprogramming peptide

<400>  18
Arg Gln Ile Lys Ile Trp Phe Gln Asn Arg Arg Met Lys Trp Lys Lys
1               5                   10                  15

Trp Ser Gln Pro Ala Ala Lys Arg Val Lys Leu Asp
            20                  25

<210>  19
<211>  33
<212>  PRT
<213>  Artificial

<220>
<223>  Reprogramming peptide

<400>  19
Gly Ala Leu Phe Leu Gly Phe Leu Gly Ala Ala Gly Ser Thr Met Gly
1               5                   10                  15

Ala Trp Ser Gln Lys Lys Arg Thr Leu Arg Lys Asn Asp Arg Lys Lys
            20                  25                  30

Arg

<210>  20
<211>  34
<212>  PRT
<213>  Artificial

<220>
<223>  Reprogramming peptide

<400>  20
Gly Ala Leu Phe Leu Gly Phe Leu Gly Ala Ala Gly Ser Thr Met Gly
1               5                   10                  15

Ala Trp Ser Gln Arg Ser Arg Lys Tyr Thr Ser Trp Tyr Val Ala Leu
            20                  25                  30

Lys Arg

<210>  21
<211>  17
<212>  PRT
<213>  Artificial

<220>
<223>  HIV gp41-derived nuclear localization signal

<400>  21
Gly Ala Leu Phe Leu Gly Phe Leu Gly Ala Ala Gly Ser Thr Met Gly
1               5                   10                  15

Ala